(19)

Europäisches Patentamt
European Patent Office
Office européen des brevets

(11) **EP 4 501 846 A1**

(12) **EUROPEAN PATENT APPLICATION**
published in accordance with Art. 153(4) EPC

(43) Date of publication:
05.02.2025 Bulletin 2025/06

(21) Application number: 23714785.5

(22) Date of filing: 23.03.2023

(51) International Patent Classification (IPC):
*C01B 32/178* [(2017.01)]  *C01B 32/18* [(2017.01)]
*A61K 51/02* [(2006.01)]  *A61P 35/00* [(2006.01)]

(52) Cooperative Patent Classification (CPC):
C01B 32/178; A61K 41/009; A61K 51/02;
A61P 35/00; C01B 32/18

(86) International application number:
PCT/ES2023/070187

(87) International publication number:
WO 2023/180615 (28.09.2023 Gazette 2023/39)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 25.03.2022 ES 202230271

(71) Applicant: Consejo Superior De Investigaciones
Científicas
(CSIC)
28006 Madrid (ES)

(72) Inventors:
• TOBIAS ROSSELL, Gerard
08193 Cerdanyola del Vallès (Barcelona) (ES)
• BATISTA GONÇALVES, Gil Alberto
08193 Cerdanyola del Vallès (Barcelona) (ES)
• SANDOVAL ROJANO, Stefania
08193 Cerdanyola del Vallès (Barcelona) (ES)

(74) Representative: Pons
Glorieta Rubén Darío 4
28010 Madrid (ES)

(54) **LITHIUM FILLED NANOCAPSULES AND USE THEREOF**

(57) The present invention relates to carbon nanocapsules (CNCs) that encapsulate a lithium isotope ($^6$Li) and to their use, preferably in neutron capture therapy and cancer treatment. The CNCs are, in particular, carbon nanohorns aggregates (CNHs) or carbon nanotubes (CNTs) with closed ends.

Fig. 1

## Description

The present invention relates to carbon nanocapsules that encapsulate a lithium isotope ($^6$Li) and to their use, mainly in neutron capture therapy. This therapy is useful for cancer treatment, among other diseases. Therefore, the present invention belongs to the field of medicine.

## STATE OF ART

Cancer is one of the leading diseases worldwide, with more than 37.5 million diagnosed cases in the last few years and high levels of incidence, prevalence, and mortality. Nowadays, several cancer treatments are available for improving the life expectancy of cancer patients, such as surgical intervention, radiation therapy and chemotherapy. However, treatment inefficiency or significant toxicity remain as the main challenges of current available oncologic therapies. Neutron capture therapy (NCT) is a high-linear energy transfer form of radiotherapy that exploits the potential of some specific isotopes for cancer treatment, based on the neutron capture and emission of short-range charged particles, which occur at low energies. The nuclear reaction that takes place when some isotopes are irradiated with low-energy thermal neutrons, produces high linear energy transfer (LET) particles suitable for cancer cell eradication. The limited path lengths of the **LET** particles (5-9 $\mu$m) produced in NCT can limit the destructive effects to isotopes that are localized in cells. Thus, conferring high therapeutic precision to this type of radiotherapy. Moreover, in therapy of deep-seated tumours, NCT currently employs epithermal neutron beams, which have higher penetration in tissues.

There is a wide number of nuclides with high affinity for the capture of thermal neutrons, consequently being potential candidates to be explored in NCT. Special attention has been paid to boron, which has been proved as one of the most promising elements for the development of active compounds for NCT. The ability of nonradioactive nuclide $^{10}$B to capture thermal neutrons resulting in the nuclear reaction $^{10}$B(n,$\alpha$)$^7$Li, has made boron neutron cancer therapy (BNCT) an option for a number of patients. The selective delivery of boron compounds in therapeutic doses to the tumour cells allows their destruction after epithermal neutron beam radiation. This treatment benefits from a large dose gradient between the tumour and normal cells thus reducing damage of the latter. To date, BNCT has been studied clinically in a variety of disease sites, including glioblastoma multiforme, meningioma, head and neck cancers, lung cancers, breast cancers, hepatocellular carcinoma, sarcomas, cutaneous malignancies, extramammary Paget's disease, recurrent cancers, pediatric cancers, and metastatic disease (Malouff T. et al. Frontiers in Oncology 2021, 11: 601820). Although there is still much room for clinical improvement, there are evidences of the potential of BNCT in treating tumours that cannot be cured with other methods due to their dissemination, infiltration, recurrence or proximity with radiosensitive tissues. Recently, clinical studies with patients with genital melanoma, considered to be radio- and chemotherapeutically resistant, showed quite impressive results (Hiratsuka J. et al. Cancer Communications 2018, 38(1): 38). All patients treated with $^{10}$B-enriched L-BPA (L-para-borophenylalanine) showed a complete local tumour control within six months, without significant side effects.

The recent technological developments that allow the installation of accelerators at medical institutions will bring NCT closer to real clinical applications (Dymova MA, et al. Cancer Communications 2020, 40: 406). However, over the years the BNCT scientific community has focused on the need of new carriers characterized by higher tumour specificity of boron compounds used as capture agents, which would result in better efficiency of the treatment and decreased risks of toxicity. Since the early days of NCT, sixty years ago, many different delivery agents have been developed and studied (He H, et al. Radiation Oncology 2021, 16: 216). However, only two pharmaceuticals have been employed in clinical trials, namely sodium mercaptoundecahydro-closo-dodecaborate (BSH; Na$_2$$^{10}$B$_{12}$H$_{11}$SH) and L-para-borophenylalanine (BPA; C$_9$H$_{12}$$^{10}$BNO$_4$) derivatives.

Several modern BNCT agents have emerged in the last few years; most of them consist of a stable boron moiety attached to a tumour-targeting unit. In this group it can be included novel polyhedral boranes, boronated nucleosides, amino acids and peptides, sugars, phospholipids, tetrapyrroles and monoclonal antibodies (Barth RF. et al. Cancer Communications 2018, 38(1): 35). However, critical issues such biodistribution and low tumour delivery obtained so far have limited the efficiency of the available molecular systems. Therefore, novel nanocarriers are currently being explored as delivery platforms in an effort to overcome the limitations of the classical delivery compounds. Gold nanoparticles capped with mercaptocarboranyl clusters or PEGylated gold nanoparticle-carborane assemblies have been postulated as potential therapeutic agents. Other types of nanoparticles, including boron phosphate, silica and boron nitride nanotubes (Nakamura H. et al. Bioorganic and Medicinal Chemistry Letters 2015, 25(2): 172-174) have also been tested. The major drawback for these nanocarriers with the boron moiety attached externally to the nanocarrier is the lack of stability under physiological conditions, which is required for (pre)clinical application of NCT. To improve the stability under physiological conditions, in 2012 a novel approach started to be explored, by designing core-shell-type biodegradable nanoparticles with boron clusters in their cores (Sumitani S, Nagasaki Y. Polymer Journal 2012, 44(6): 522-530). The obtained core-polymerized and boron-conjugated micelles showed high stability under physiological conditions and a high accumulation in tumours. In the case of liposomal formulations, an increase in the stability can be obtained if the boron species are incorporated into the internal cavity of the host material. This approach is starting to be more exploited; for example, BSH-encapsulating 10 % distearoyl boron lipid liposomes have been developed (Koganei H. et al. Bioconjugate Chemistry 2013, 24(1): 124-132). Liposomes have high boron content (B/P ratio 2.6) and display excellent delivery efficacy. Theranostic nanoplatforms based on carborane-containing a cholesterol derivative have been explored for BNCT treatment of mesothelioma, monitored in real-time by MRI (Alberti D. et al. Organic and Biomolecular Chemistry 2014, 12(15): 2457-2467). *In vivo* tests performed with the new theranostic agents, revealed that after irradiation with thermal neutrons, the tumour masses showed a drastic reduction of about 80-85 %.

With decrease of expectancies over the efficiency of boron compounds as NCT active agents, other elements have been attempted. The high cross section value of $^{157}$Gd (over sixty times higher than $^{10}$B; 254 000 b for $^{157}$Gd vs 3800 b for $^{10}$B) and its paramagnetic behaviour, makes it an outstanding candidate for the development of multifunctional NCT agents able to be trackable by MRI imaging (Gadolinium Neutron Capture Therapy - GdNCT) (Enger SA. et al. Radiation Measurements 2013, 59: 233-240). However, therapeutic efficiency of $^{155/157}$Gd is significantly smaller than $^{10}$B, because the interaction with thermal neutrons leads to the emission of Auger electrons with short range in tissues, thus cytotoxic effects can be obtained only if Auger are generated in the proximity of DNA in order to induce double-strand breaks (Martin RF. et al. Pigment Cell Research 1989, 2(4): 330-332).

For example, cellular studies of Gd-DTPA or Gd-DOTA drugs showed penetration and accumulation of 84 % and 56 % in the cell nuclei, respectively. However, these values decrease drastically for the studies of Gd-DTPA performed in human glioblastoma multiforme (GBM) tumours (6.1 %) (De Stasio G. et al. Neurological Research 2005, 27(4): 387-398). Recently, Lee *et al.* explored functionalized $Gd_2O_3$ nanoparticles as potential NCT theragnostic agents (Ho SL. et al. RSC Advances 2018, 8(23): 12653-12665). Besides the improved T1 magnetic resonance imaging (MRI) performance of the contrast agent, the *in vitro* studies exhibited low values of U87MG tumour cell death (28.1 %) after thermal neutron beam irradiation (1.75 times higher than that obtained using commercial *Gadovist)*. Several preclinical investigations performed based on gadolinium compounds revealed very frustrating results, leading to the almost complete abandonment of this GdNCT approach by the scientific community. The poor selective delivery, low water solubility and the low therapeutic effectiveness of the gadolinium drugs were proposed as main reasons for GdNCT failure.

Recently, relevant technological improvements have been made in terms of accelerators as a suitable neutron source for NCT. However, low selective delivery of current dugs to cancer cells remains as the main challenge for successful clinical application of NCT.

With the aim of solving the inconveniences of the prior art, new carbon nanocapsules, filled with a neutron capture element, have been developed that significantly improve the efficiency of NCT in medical applications. The nanocapsules, of several nanometers in diameter, hermetically seal a high density of the neutron capture element and prevent its direct interaction with the biological media. The presence of functional groups on the external aromatic structure of the nanocarbon shelter confers good water dispersibility and enhance its biocompatibility.

## DESCRIPTION OF THE INVENTION

The present invention presents an innovative and previously unexplored approach for the design of novel nanotherapeutic NCT agents. Herein, a new concept based on carbon nanocapsules that hermetically seal $^6$Li active NCT nuclides has been developed. The $^6$Li active species are located in the inner cavity of the carbon nanocarrier and therefore, completely protected from the biological environment, avoiding toxicity and degradation. After encapsulation of the active cargo, the external surface of the nanocarrier has been modified for improved biocompatibility. The developed nanocapsules offered the possibility to use $^6$Li compounds as active NCT agents by delivering therapeutic doses to cancer cells. The inventors have envisaged that nanoencapsulation of $^6$Li into hermetic nanocapsules can trigger the successful development and implementation of Lithium Neutron Cancer Therapy (LiNCT). Additionally, the NCT agents described herein could be used in other types of therapy, such as neutron capture enhanced particle therapy (NCEPT), which involves injecting a neutron capture agent into a patient immediately before proton or heavy ion therapy is administered.

The possibility to encapsulate a high density of $^6$Li nuclides in the form of inorganic compounds, plus the fact that nuclear reactions of $^6$Li resultant from neutron irradiation produce higher energetic particles than the ones resultant from $^{10}$B, can significantly improve the efficiency of NCT in the eradication of cancer cells.

Then, a first aspect of the present invention relates to a carbon nanocapsule (CNC) encapsulating lithium isotope $^6$Li and wherein said nanocapsule is selected form carbon nanohorns aggregate (CNHs) and closed-ended carbon nanotube (CNT).

The term "carbon nanotube (CNT)" refers to one of the allotropes of sp$^2$ hybridized carbon characterized by cylindrical elongated shape and nanometric diameter. CNTs are made of rolled-up honeycomb carbon lattice of graphene. According to the number of graphene layers, carbon nanotubes are classified as single- or multi-walled nanotubes (denoted as SWCNTs and MWCNTs).

For the purposes of the present invention, the term carbon nanotube (CNT) includes single-wall carbon nanotube (SWCNT) as well as multi-wall carbon nanotube (MWCNT).

The term "carbon nanohorns aggregate (CNHs)" refers to an aggregate (spherical cluster or bundle) of tiny graphene sheets, wrapped to form horn-shaped cones with a half fullerene cap, having typically 30-50 nm length and 2-5 nm average diameter. The aggregate could have different morphologies like "dahlia" flowers or buds, with an overall diameter (i.e.: larger dimension, which refers to the longest distance between two tips of the CNHs) that ranges between 50-120 nm. The indicated values of length, diameter and overall diameter refers to average values, as measured by SEM. These CNHs can be acquired from the supplier *Carbonium srl,* Padua-Italy.

In a preferred embodiment, the CNC is a dahlia type aggregate of CNHs.

The CNC of the present invention are CNHs aggregates as defined above, which have closed tips, or closed-ended carbon nanotubes. That is, the carbon nanomaterials have their ends or tips closed, forming a sealed capsule with an inner cavity containing the lithium isotope $^6$Li. This hermetically sealed carbon capsule isolates the encapsulated compound from the external medium, which is an essential aspect for the development of CNC for biomedical purposes.

In a preferred embodiment, the length of the closed-ended carbon nanotubes is between 80 and 300 nm, and the external diameter of said tubes ranges from 1 to 30 nm. Preferably, in the case of MWCNTs, the external diameter ranges from 5 to 30 nm and in the case of SWCNTs, the external diameter ranges from 1 to 2 nm, as determined by electron microscopy (TEM or SEM), preferably, determined by TEM (transmission electron microscopy). The indicated length and diameter values refer to average values.

In a preferred embodiment, the diameter (i.e.: larger dimension) of the CNHs ranges from 50 to 120 nm as determined by scanning electron microscopy (SEM). The indicated values refer to average values (average diameter).

Despite both materials, CNTs and CNHs, are carbon allotropes with graphene-based walls, they present different morphology. CNTs have a cylinder shape with only two ends whereas CNHs present a dahlia-like shape with a large number of tips susceptible for their filling.

In a preferred embodiment, the concentration of lithium isotope $^6$Li in the carbon nanocapsule ranges from 0.7 at. % to. 10 at. %, more preferably, between 0.7at.% and 1.1 at.%, as determined by XPS analysis.

In a preferred invention, the carbon nanocapsules comprise no other neutron capture element than $^6$Li.

The term "neutron capture element" means a chemical isotope which, when irradiated by neutrons, captures them in its nucleus and emits electromagnetic energy and/or particles.

In a preferred embodiment, lithium isotope $^6$Li is in the form of a lithium salt, preferably selected from: $^6$LiCl, $^6$LiF, $^6$LiI, $^6$LiNO$_3$, $^6$Li$_2$SO$_4$, $^6$Li$_2$CO$_3$, more preferably $^6$LiI.

The external surface of the carbon nanocapsules can be functionalized to improve their water dispersibility, biocompatibility and cell internalization.

In a preferred embodiment, the external surface of the carbon nanocapsules is functionalized with a group selected from: amine, carboxylic acid, hydroxyl, dextran, pluronic acid, polyethylene glycol (PEG) and albumin.

More preferably, the external surface is functionalized with free amine (-NH$_2$) groups.

The quantity of these groups in a carbon nanocapsule ranges from ca. 1 to 8 mmol·g$^{-1}$.

Another aspect of the present invention refers to the process for preparing the carbon nanocapsules described in the first aspect of the present invention.

The carbon nanocapsules of the present invention can be prepared from CNTs, preferably MWCNTs, or CNHs aggregates, which are commercially available. In addition, these starting materials can be prepared following the methods described in the prior art *(*Sinnott S. et al. Critical Reviews in Solid State and Materials Sciences 2001, 26: 145 for CNT and Liu X. et al. Biosensors and Bioelectronics 2020, 167: 112495 for CNH).

The process for preparing the carbon nanocapsules (CNCs) described in the first aspect of the present invention comprises, from CNCs having open ends/tips, the encapsulation of $^6$Li and closing their open ends/tips by annealing at a temperature between 700 and 1300 °C a mixture of CNCs with a $^6$Li salt in a weight ratio CNCs: $^6$Li salt from 1:5 to 1:20 for 4-12h.

In a preferred embodiment, the annealing step is carried out in a weight ratio CNCs: $^6$Li salt of 1:10.

After the encapsulation and closing step, external surface functionalization of the CNCs can be carried out. Depending on the type of functionalization to be included, one reagent will be used or the other. For example, to include an amine (-NH$_2$) functionalization on the surface, the CNCs encapsulation $^6$Li are treated with diazonium-based arylation reaction as known in the prior art (Georgakilas V. et al. Journal of the American Chemical Society 2002, 124(5): 760-761).

The starting material to prepare CNCs according to the present invention are preferably commercially available CNTs or CNHs. These commercially available products usually have their ends or tips closed, so that it is necessary to open them to carry out the encapsulation step.

Then, in a preferred embodiment of the process of the invention, the CNCs are CNTs, and the process comprises, before the encapsulation and closing step, the steps of:

- oxidation of closed-ended CNTs to open their ends by treating them with a strong oxidizing medium: nitric acid or a mixture of nitric:sulphuric acids for 6-15 h.
- thermal reduction of the CNTs, obtained in the previous step, at 500-800 °C for 1-2 h under vacuum.

After the thermal reduction of the CNTs, the encapsulation and closing step described above is carried out.

The acid treatment (oxidation step) is a simple and efficient approach that allows cutting CNTs, removes metal particles (catalyst employed for the growth of CNTs) and induces the formation of O-containing moieties ($sp^3$ defects) along the CNTs surface.

The thermal reduction step allows to remove the functionalities introduced on the graphitic structure of the CNT by the acid treatment. This step is performed to avoid secondary reactions with the melted lithium salts during the filling step.

In a preferred embodiment, the thermal reduction is carried out at 800 °C for 2 h.

In a preferred embodiment, the annealing step is carried out at a temperature between 1000 and 1300°C, more preferably at 1100°C.

In a preferred embodiment of the process of the invention, the CNCs are CNHs and the process comprises, before the encapsulation and closing step, the step of: oxidation of the CNHs to open their tips by annealing them up to 400-550 °C under air.

After the oxidation of the CNHs, the encapsulation and closing step described above is carried out. In a preferred embodiment, the annealing for the encapsulation and closing the opened ends/tips is carried out at a temperature between 700 and 1100°C, more preferably at 1100°C.

Another aspect of the present invention refers to the CNCs defined in the first aspect of the present invention for use as a medicament.

In a preferred embodiment, the invention refers to CNCs defined in the first aspect for use in the diagnosis or treatment of cancer.

In a preferred embodiment, the invention also refers to CNCs defined in the first aspect for use in neutron capture therapy (NCT), more preferably, in the treatment of cancer by NCT. Said CNCs act as neutron capture therapy agents.

As the iodine is a contrast agent, the CNCs of the present invention can be used in diagnosis, for example using X-ray computed tomography (XCT)

Although the CNCs described in the present invention have their main application in NCT, their use in other types of therapies, such as, for example, Neutron capture enhanced particle therapy (NCEPT), is not discarded.

In consequence, the present invention also refers to CNCs defined in the first aspect for use in NCEPT, that entails injecting a neutron capture agent into a patient shortly before irradiation with proton or heavy ion treatment.

The CNCs described in the present invention present notable advantages with respect to the NCT agents known in the state of the art. These advantages are summarized below:

- Hermetic sealing of a large amount of neutron capture atoms ($^6$Li) per each nanocarrier. This avoids toxicity and direct interaction with the biological milieu.
- Ease to functionalize the external surface of the nanocapsules improving in this way the water dispersibility, biocompatibility and cell internalization.
- Deliver high doses of $^6$Li therapeutic agents to cancer cells, able to promote their cell death after neutron irradiation.
- The use of nanocapsules for the delivery of $^6$Li present several advantages with respect to the use of molecular entities, including a prolonged circulation time in blood and the possibility to be accumulated in the diseased site by both active and passive targeting.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

## BRIEF DESCRIPTION OF THE DRAWINGS

Fig. 1. Electron microscopy characterization of the nanocarriers. TEM images of (a) acid treated/centrifuged (20000 rpm) MWCNT and (c) thermally treated CNHs (500 °C) under oxidizing atmosphere. (b, d) show the histograms with the size distribution of the respective carbon nanostructures determined by SEM. Arrows point to open-ended CNTs.

Fig. 2. Z-contrast STEM and HRTEM images of (a, b and c) $^6$LiI@MWCNTs and (d, e and f) $^6$LiI@CNHs, prepared by molten phase filling.

Fig. 3. a) Qualitative neutron autoradiography of the KBr pellet containing [6]LiI@MWCNTs. b). Quantitative distribution of lithium in a portion of the sample.

Fig. 4. High-resolution spectra over C1s, N1s and I3d for functionalized CNCs: $NH_2$_[6]LiI@MWCNT and $NH_2$_[6]LiI@CNH. Peak fitting was performed using Origin software employing Gaussian curves.

Fig. 5. Qualitative neutron autoradiography of a cell pellet exposed to $^6$Li@CNC produced by CNHs a) and MWCNTs b).

Fig. 6. SEM images of MWCNTs after the employed acid treatment followed by centrifugation at a) 4000 rpm and c) 20000 rpm, b) and d) show the respective length distribution histograms obtained by measuring over 1000 individual MWCNTs.

Fig. 7. AFM images of acid treated and centrifuged a) MWCNTs (20 000 rpm) and b) CNHs after the oxidizing treatment (500 °C).

Fig. 8. Thermogravimetry analysis under flowing air of as-received MWCNTs, after oxidizing acid/centrifuge treatment and after the reduction treatment under vacuum at 800 °C during 2 h.

Fig. 9. Specific surface area determined by BET analysis for as-received and thermally treated CNHs in air at a heating rate of 1°C·min$^{-1}$ until 400 °C (CNHs400°C) or 500 °C (CNHs500°C).

Fig. 10. Thermogravimetry analysis under flowing air of as-received CNHs, and thermal treated at 400 °C and 500 °C.

Fig. 11. a) SEM image of CNHs after the oxidizing treatment (500 °C), and b) respective length distribution histograms obtained by measuring over 500 individual CNHs.

Fig. 12. HRTEM images of [6]LiI encapsulated into MWCNTs. Annealing treatments lead to the simultaneous filling and end-closing of the CNTs (carbon nanocapsules).

Fig. 13. Energy Dispersive X-Ray analysis of [6]LiI@MWCNTs. The inset corresponds to the area of the sample where the data was acquired.

Fig. 14. Energy Dispersive X-Ray analysis of the sample [6]LiI@CNHs. The inset corresponds to the sample where the data was acquired.

Fig. 15. High resolution XPS spectrum of I3d for sample a) [6]LiI@MWCNTs and b) [6]LiI@CNHs.

Fig. 16. Schematic representation of the surface functionalization by cycloaddition of CNCs made on CNHs.

EXAMPLES

Next, the invention will be illustrated by some examples carried out by the inventors in order to demonstrate the effectiveness of the product of the invention.

Example 1: Preparation of CNCs from MWCNTs and CNHs:

1.1. Purification, shortening and tip-opening of MWCNTs

The raw MWCNTs used in this work were acquired from NanoAmor (Short MWNT (95+%, OD 20-30 nm), Product ID 1237YJS). The oxidative cutting of the MWCNTs was performed using a combination of acid treatment and ultrasonication, as previously reported (Kierkowicz M. et al. Carbon 2018, 139: 922-932). Briefly, 150 mg of MWCNTs were dispersed in 150 mL of a mixture of $H_2SO_4$/$HNO_3$ (3/1) and sonicated during 24 h at a temperature of ca. 40 °C. The resulting suspension was washed with distilled water by vacuum filtration using a 0.2 $\mu$m PTFE membrane (omnipore), until the filtrate reached neutral pH. After that, the MWCNTs were dispersed again in ultrapure water (200 mL) by ultrasonication, and then centrifuged at either 4000 or 20000 rpm during 30 min. The precipitate was discarded (bundles or long nanotubes), and the process was repeated twice. The MWCNTs suspension was collected by vacuum filtration and dried in the oven overnight at 100 °C. Finally, the prepared short MWCNTs were reduced by thermal treatment in order to remove oxygen functional groups introduced during the shortening step. For this purpose, short-MWCNTs (50 mg) were inserted in a silica tube and the sample was left under vacuum for 2 h. The tube was then placed inside a furnace and annealed at 800 °C under vacuum during 2 h (heating rate 200 °C·h$^{-1}$).

## 1.2. Oxidizing thermal treatment of CNHs for opening the tips

CNHs aggregate were acquired from Carbonium (SWCNH dahlia type). The CNHs were thermally treated at two different temperatures under oxygen atmosphere ($O_2$) in order to oxidize the carbon tips allowing in this way full access to their inner cavity, following the protocol described by Utsumi *et al.* (Utsumi S, et al. The Journal of Physical Chemistry B 2005, 109(30): 14319-14324). Briefly, 50 mg of CNHs were placed in a silica boat and then placed in a tubular furnace. The thermal treatment was carried out under dry synthetic air, reaching a maximum temperature of 400 or 500 °C (heating rate of 1°C·min$^{-1}$). The samples were immediately cooled down after reaching the maximum temperature.

## 1.3. Synthesis of $^6$Li@CNC

The filling of MWCNTs was attempted with different lithium compounds via molten capillarity wetting, following a modified protocol from the one reported by Sloan *et al.* for the encapsulation of LiI into single-walled CNTs (Brown G. et al. Applied Physics A 2003, 76(4): 457-462). Briefly, 10 mg of shortened MWCNTs were mixed with 100 mg of the selected lithium compound inside an Ar filled glovebox (using an agate mortar and pestle), until the sample presented a homogenous colour. Subsequently, the powder was sealed under vacuum in a silica tube of ca. 10 cm in length. The silica ampoule was placed into a furnace and the mixture was annealed at 5 °C·min$^{-1}$ until reaching the first dwell step (50 °C above the melting point of the corresponding salt: LiI, LiCl and LiF acquired from Sigma-Aldrich with purity > 99 %. After 4 h, the temperature was increased (5 °C·min$^{-1}$) up to 1100 °C, where the system was dwelled for 10 min. Finally, the sample was cooled down (1 °C·min$^{-1}$) until a temperature 50 °C above the melting point of the inorganic salt and subsequently the cooling rate was increased to 5 °C·min$^{-1}$ until room temperature. This thermal treatment was designed to allow the formation of filled closed-ended CNTs in a single step (Martincic M. et al. Carbon 2019, 141: 782-793). For the filling of CNHs with lithium salts, slight variations to the annealing procedure were applied. The silica ampoule (sealed under vacuum), containing the corresponding mixture (lithium salt/CNHs) was placed inside the tubular furnace and then annealed at 950 °C for 12h. The system was subsequently cooled down to 600 °C at 1°C·h$^{-1}$ and finally to room temperature at 5 °C·h$^{-1}$. The inorganic salt that showed high filling rate of CNCs (MWCNTs as well as CNHs) was also explored in its enriched form, $^6$LiI (Sigma-Aldrich, purity > 99 %).

After the filling step, CNCs were purified by washing the excess of inorganic material from the external surface. The mixture was first dispersed in water (sonication for 15 minutes) and then refluxed at 110 °C during 4h. This purification cycle was repeated three times, in order to guarantee the complete removal of the excess of non-encapsulated lithium compounds. The purified CNCs were collected by vacuum filtration using a 0.2 $\mu$m PTFE Membrane (omnipore). Afterwards, the sample was dried overnight at 100 °C.

## 1.4. External surface functionalization of $^6$Li@CNC

The external surface functionalization of the CNC with free amine groups was performed using diazonium-based arylation reaction (Georgakilas V. et al. Journal of the American Chemical Society 2002, 124(5): 760-761). First, the CNC (20 mg) were dispersed in DMF (20 mL) by ultrasonication during 30 min. After that, 200 mg of 4-[(N-Boc)aminomethyl] aniline were added to the dispersion and dissolved by mechanical stirring. The mixture was cooled down to 0 °C and then 200 $\mu$L of isopentyl nitrite were added. The reaction temperature was raised to 80 °C and kept stirring during 12 h. The sample purification was performed by vacuum filtration using the following sequence of solvents: DMF/water/methanol/diethyl ether. Finally, the sample was dried under vacuum overnight.

The N-Boc functional group deprotection at the surface of the CNC was performed by treatment with a 4 M solution of dioxane (in HCl). The resulting CNC were initially well dispersed in 20 mL of dioxane solution by ultrasonication during 15 min and then, the dispersion was kept stirring overnight at room temperature. The sample purification was performed by vacuum filtration using the following sequence of solvents: water/methanol/diethyl ether. Finally, the samples were dried under vacuum overnight.

## 1.5. Characterization

Samples for microscopy characterization were prepared by placing dropwise onto a holey carbon grid. The suspension was prepared by sonicating a small amount of CNC in absolute ethanol. Transmission electron microscopy (TEM) images were obtained using a JEOL 1210 microscope, operating at 120 kV. HRTEM, HAADF and STEM were carried out in a FEI, Tecnai G2 F20 microscope operated at 200 kV. Scanning Electron Microscopy (SEM) imaging was acquired using a Quanta FEI 200 ESEM FEG microscope operating at 5 kV.

Atomic force microscopy (NanoScope IV SPM Controller, Veeco) in tapping mode was used for surface topography studies of the different carbon nanostructures deposited on a mica substrate.

Brunauer-Emmett-Teller (BET) analysis was performed on 15-30 mg of raw CNHs afther being annealed at 400 °C or 500 °C dry air. The samples underwent a treatment of adsorbed gas removal at 150 °C for 2 h. This was followed by nitrogen adsorption/desorption.

Thermogravimetric analyses were performed on a Netzsch instrument, model STA 449 F1 Jupiter®, under flowing air at a heating rate of 10 °C.min-1.

Samples for XPS were prepared by placing dropwise a dispersion prepared by sonicating the sample in dry ethanol onto a silicon wafer (5 mm x 5 mm, previously washed in water/acetone). XPS measurements were performed at room temperature with a SPECS PHOIBOS 150 hemispherical analyzer (SPECS GmbH, Berlin, Germany) in a base pressure of $5 \times 10^{-10}$ mbar using monochromatic Al Kalpha radiation (1486.74 eV) as excitation source. High resolution spectra of C1s, O1s, N1s and I3d regions were also registered.

## Example 2: Neutron irradiation experiments

### 2.1. Sample preparation for neutron irradiation

Neutron irradiation experiments with $^6$LiI@CNC were performed using KBr pellets. The pellets were prepared by grinding 200 mg of KBr with 2 mg of $^6$LiI@CNC using an agate mortar and pestle. The resulting mixture was pressed (5 ton) using a mould pellet press die with a diameter of 1.0 cm. These pellets were prepared to study the presence and the uniformity of lithium in nanomaterials, by means of qualitative autoradiography (see below).

### 2.2. Cell preparation and treatment with $^6$Li@CNC

To evaluate the uptake, rat osteosarcoma UMR-106 cell line was employed, as extensive experience of BNCT has been acquired using this model (Bortolussi S. et al. Radiation Oncology 2017, 12(1): 130). Cells were cultured in medium composed by DMEM high glucose, 10 % FCS and 1 % gentamicine. Then seeded at the density of $3 \times 10^6$ cells in two 75cm$^2$ flasks. After 48 hours, the culture medium was replaced and cells were allowed to grow for 4 hours in the medium enriched with the carbon nanomaterials containing lithium. The carbon nanomaterials $^6$Li@CNC contained in each vial were diluted in 20 ml of culture medium, hand shacked until dissolved and then ultrasonicated for 30 minutes. Before adding the enriched cell culture medium to the cell culture flasks, the enriched medium was exposed to UV light for 15 minutes to avoid cell contamination. At the end of the fixed contact time, after the medium removal, the cells were washed five times in PBS, trypsinized and counted. Finally, four million cells were selected and centrifuged, forming a condensed cell pellet. The pellet was then deposited on a mylar disk and left drying.

### 2.3 Neutron Autoradiography

Neutron autoradiography is based on the use of passive detectors sensitive to the charged radiation. Samples containing elements capturing neutrons and emitting charged particles are irradiated in contact with the detector. After irradiation the detector is etched in a chemical solution which enlarges the latent tracks and makes them visible for microscopical analysis. Depending on the calibration of the technique (neutron fluence, etching parameters), it can be employed to obtain quantitative information on the element concentration in a sample or qualitative imaging of its spatial distribution. Quantitative neutron autoradiography was used to evaluate the lithium uptake in cell samples, by detecting alfa tracks produced with the thermal neutron capture reaction on $^6$Li:

$$n + {}^6_3Li \rightarrow {}^4_2He \ (2.05 \ MeV) + {}^3_1H \ (2.73 \ MeV)$$

The dry cell samples on the mylar disks were irradiated two times in the termal neutron column of the TRIGA MARK II nuclear reactor at Laboratori Energia Nucleare Applicata (LENA) of the University of Pavia. For the first irradiation, samples were placed on a CR39 and irradiated for 30 minutes at 2 kW reactor power, with a total thermal neutron fluence of $1.97 \pm 0.01 \times 10^{10}$ cm$^{-2}$. This neutron autoradiography set-up is sensible to alpha particles, and can perform quantitative autoradiography analysis by counting tracks generated by alpha particles (Postuma I. et al. Reports of Practical Oncology & Radiotherapy 2016, 21(2): 123-128). The results are presented as a composition of 40 (8x5) sequential pictures. Each image having an area of 0.3 mm$^2$ (0.632 mm x 0.474 mm), giving a total measured area of 12 mm$^2$, which is a representative portion of the entire sample.

Qualitative images were obtained both for cell samples and for KBr pellets with a second irradiation. Samples were placed on another CR-39 and irradiated in the same position for 2 hours at 250 kW. A higher neutron fluence cause a higher track density on the detector, thus allowing the formation of a map of Li distribution in the samples (Altieri S. et al. Applied Radiation and Isotopes 2008, 66(12): 1850-1855). Qualitative autoradiography results in a black and white picture, where the gray halo represents the region with lithium uptake. The whiter parts of the images are the ones with higher lithium concentration.

### 2.4. *In vitro* neutron irradiation experiments

NCT effects were studied using the same cell line described above. To evaluate the effect of lithium due to neutron irradiation, four flasks were prepared: 2 serving as the control, without drug treatment (non-enriched cells) and 2 where cells were exposed to the $^6$Li@CNC drug (enriched cells). Rat osteosarcoma UMR-106 cells were cultured in medium composed by DMEM high glucose, 10 % FCS and 1 % gentamicine. Then seeded at a density of $3 \times 10^6$ cells in four 75cm$^2$ flasks. After 48 hours, for two flasks, the culture medium was replaced and cells were allowed to grow for 4 hours in the medium enriched with the carbon nanomaterials containing lithium, following the same procedure as described in Section 2.2. At the end of the fixed contact time, cells were washed and fresh medium was added to the flask. The same procedure was performed for the other two control flasks. Finally, a non-enriched and an enriched cell flask were irradiated in the thermal column of the TRIGA Mark II reactor for 11 minutes at 250 kW. The irradiation position is characterized by a thermal neutron flux of about $10^{10}$ cm$^{-2}$ s$^{-1}$ at 250 kW. Cell survival was evaluated by means of cloning assay, measuring the plating efficiency.

### Results and discussion of the above-described examples 1 and 2.

### Preparation of CNC from MWCNTs and CNHs

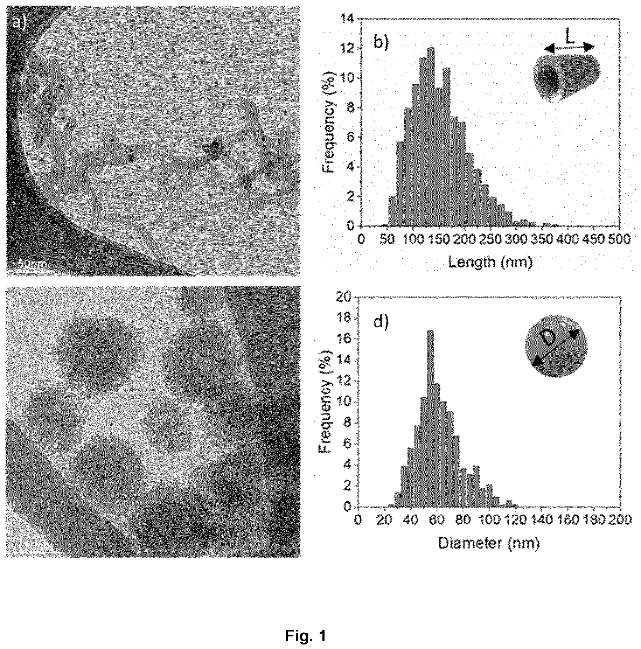

As indicated previously, two different types of carbon nanostructures, namely MWCNTs and CNHs were explored as nanocarriers for the delivery of enriched $^6$Li into cancer cells. The inventors have noticed that the preparation of viable carbon nanomaterials for melt filling is dependent on the employed carbon nanostructure. In the case of MWCNTs, a two-step treatment was usually needed for the preparation of nanocapsules. The first step consists in an oxidizing protocol, which combines a mixture of $H_2SO_4$/$HNO_3$ and ultrasonication. Acid treatment is a simple and efficient approach that allows cutting MWCNTs, removes metal particles (catalyst employed for the growth of CNTs) and induces the formation of O-containing moieties (sp$^3$ defects) along the CNTs surface. With the aim to discard long CNTs or bundles that can still be present after the oxidative cutting, the inventors took advantage of the water dispersibility of the acid treated MWCNTs to fractionate them by length via centrifugation (at 4000 and 20000 rpm). The precipitated material at the bottom of the centrifuge tube was discarded and the supernatant was collected. In this way, purified, open-ended and very short MWCNTs with a narrow length distribution were obtained (Figure 1 a).

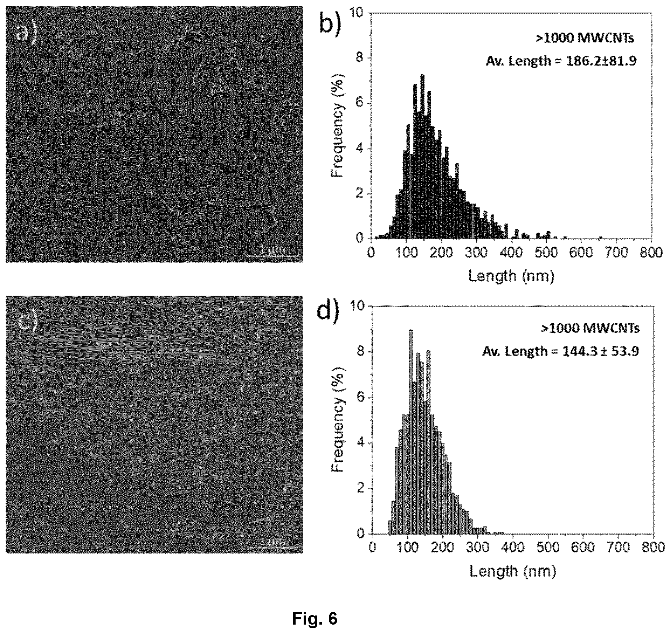

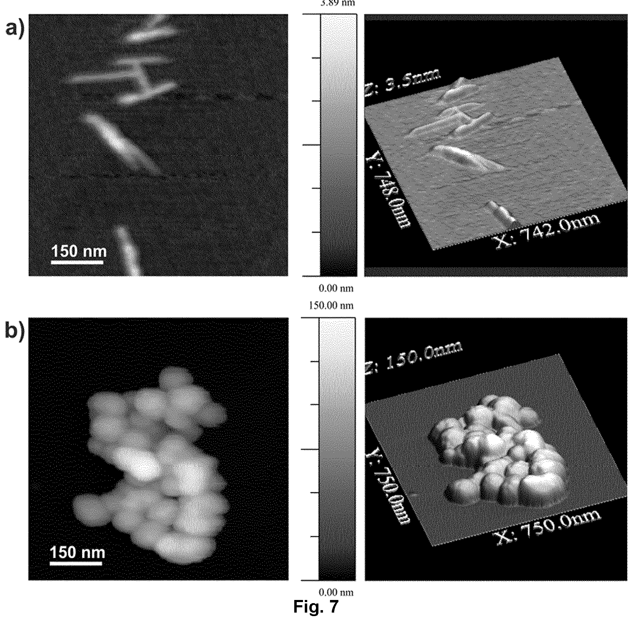

Fig. 6 shows the length distribution of the acid cut MWCNTs after applying different centrifugation speeds, 4000 and 20000 rpm. The length distribution was determined by measuring over 1000 specimens from SEM images. Statistical analysis reveals an average length distribution of $186.2 \pm 81.9$ and $144.3 \pm 53.9$ nm for the MWCNTs centrifuged at 4000 rpm (Fig. 6 a) and b)) and 20000 rpm (Fig. 6 c) and d)) respectively. Therefore, due to the small average size and narrow distribution, MWCNTs obtained at the highest centrifugation speed were employed in subsequent studies. It is important to notice that the obtained length distributions are within the range of improved biocompatibility for CNTs (Serpell CJ. et al. Nature Communications 2016, 7: 13118). AFM imaging corroborates the very short length of MWCNTs observed by SEM and gives an additional overview of the morphology of the samples (Fig. 7). Subsequently, the short MWCNTs were thermally reduced in order to remove the functionalities introduced on their graphitic structure by the acid treatment. This step is performed to avoid secondary reactions with the melted lithium salts during the filling step. The elimination of the aliphatic fractions was confirmed by thermogravimetric analysis (TGA,

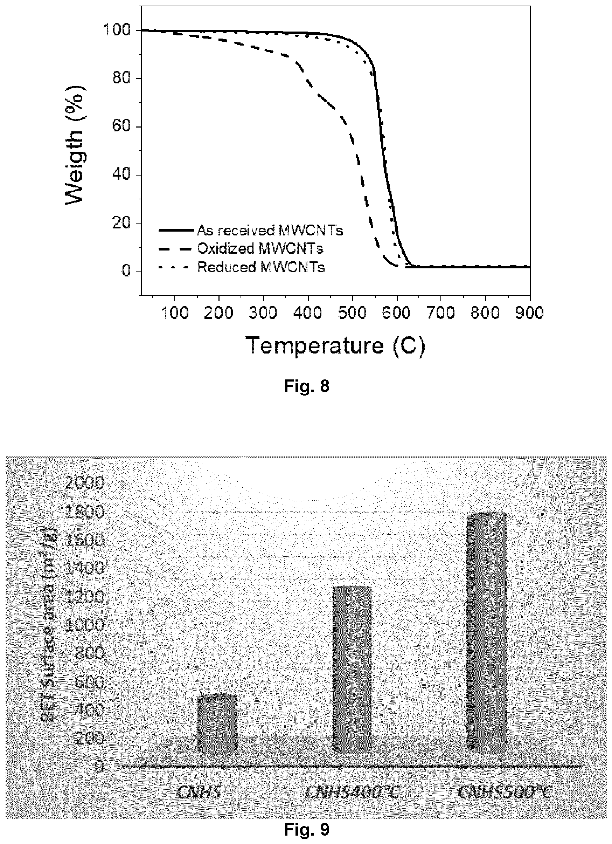

Fig. 8. Whereas the TGA curve of the acid treated MWCNT shows a continuous weight loss due to the removal of adsorbed water and functional groups (red line), the vacuum annealed sample becomes thermally stable until its complete combustion at ca. 550 °C (green line).

The preparation CNHs suitable for the synthesis of CNC is much simpler and only requires a single oxidizing step for the controlled tip hole opening of their carbon structure. This controlled thermal oxidation can be applied because disruption of CNHs preferentially occurs through the tips, where a high density of structural defects or high strain is commonly present. As received CNHs were independently treated at 400 °C and 500 °C in air. The collected samples were analysed by BET to determine the specific surface area (SA), that allows to estimate the degree of tip opening at each treatment temperature. Comparison of the SA of raw CNHs (CNHs= 419 m$^2$ g$^{-1}$) and after being annealed at 400 °C (CNHs$_{400°C}$ =1317 m$^2$ g$^{-1}$) and 500 °C (CNHs$_{500°C}$= 1857 m$^2$ g$^{-1}$), reveals the impact that temperature has on the degree of tip opening (Fig. 9). The largest surface area is reached at the highest employed temperature. The value of specific surface area achieved for CNHs$_{500°C}$ is significantly higher than previously reported by Fan *et al.* 1450 m$^2$·g$^{-1}$.( J. Phys.

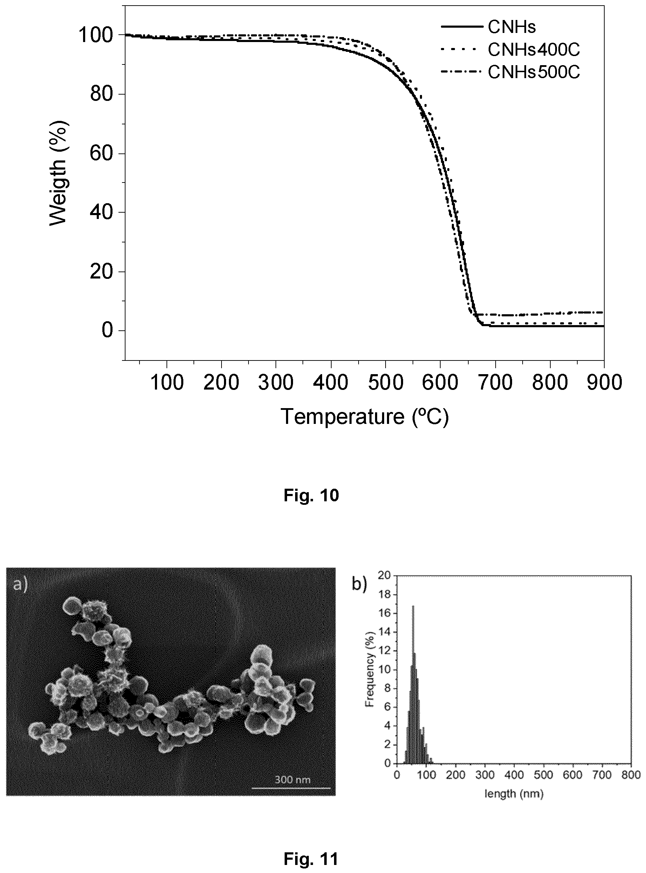

Chem. B 2006, 110, 4, 1587-1591) It almost reaches the value achieved for graphene (Wang JT-W. et al. Carbon 2020, 162: 410-422), showing that both sides of the single carbon layer in CNHs are fully accessible (Martincic M. Tobias G. Expert Opinion on Drug Delivery 2015, 12(4): 563-581). Additional characterization using TGA, under flowing air, reveals that both thermally treated CNHs present a similar combustion curve to as-received CNHs (Fig. 10). This indicates that the controlled oxidation at 400°C and 500°C takes only place in the tips and preserves the structural integrity of CNHs. Consequently, the CNHs with highest SA were selected for the development of nanocapsules. The star-like carbon structure of CNHs allows to obtain a large number of opened tips, it is expected to maximize the filling rate with the lithium compounds.

Figure 1b shows a TEM image of CNHs after the oxidizing thermal treatment at 500 °C. Statistical analysis reveals a median diameter of 59.3 $\pm$ 16.9 nm determined by measuring over 500 specimens from SEM images. AFM and SEM images of CNHs are shown in Fig. 7 and Fig. 11.

## Encapsulation of $^6$Li onto CNC ([6]LiI@CNC)

To maximize the lithium loading in short MWCNTs, via molten phase capillary wetting, several lithium salts were attempted, namely lithium iodide, lithium chloride and lithium fluoride. These salts were independently ground with a given amount of MWCNTs and sealed under vacuum in a silica ampoule. The mixture was then heated above the melting temperature of the salt to allow capillary wetting of the inner cavities of the open-ended MWCNTs by the molten halide. The obtained results showed that the high reactivity of lithium chloride and lithium fluoride with the silica ampoules at the temperatures employed for the filling step restrict their use in this approach. Consequently, only lithium iodide ([6]LiI) was explored on this work for the preparation of CNC. In general bulk filling of carbon nanomaterials results in the presence of a large amount of non-encapsulated compounds (Kierkowicz M. et al. ACS Sustainable Chemistry & Engineering 2017, 5(3): 2501-2508). Therefore, after the filling experiment, [6]LiI that remained external to the MWCNTs or CNHs was removed from the sample by performing several washings in water (LiI solubility = 1.67 g· g$^{-1}$ at 25 °C).

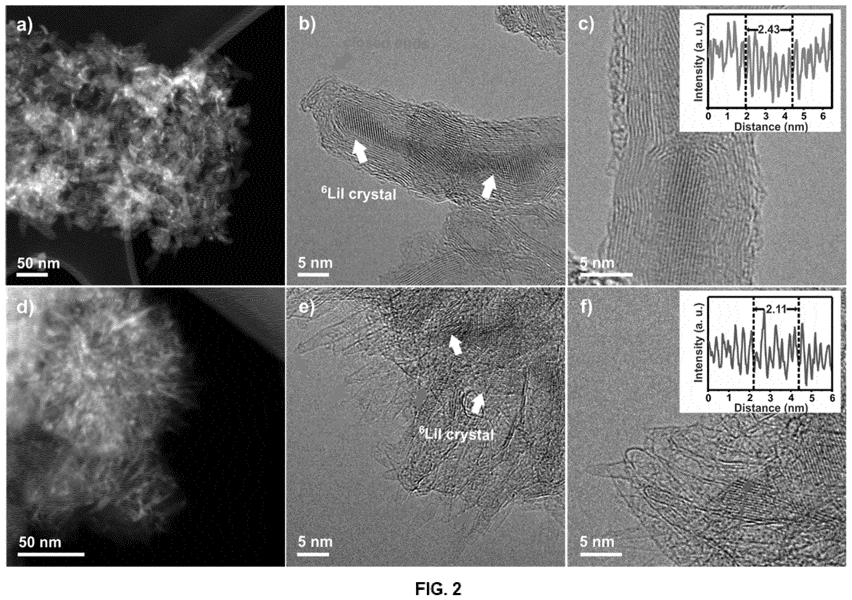

The successful filling of MWCNTs and CNHs with [6]LiI was initially determined by Z-contrast imaging. Due to significative difference in the atomic number of carbon and iodine from the filled salt, remarkable contrast differences between the components were detected when performing high-angle annular dark field (HAADF) imaging in high resolution scanning transmission electron microscopy (STEM). Thus, confirming the presence of [6]LiI in the inner cavity of MWCNTs and CNHs (Figure 2a and 2d, respectively). Indeed, the homogenous distribution of small fragments of bright straight lines inside the darker carbon shells confirms the efficiency of the employed methodology for the preparation of the CNC. Besides, no evidence of bright particles was observed outside the carbon nanostructures, confirming the full removal of the non-encapsulated [6]LiI. The selective dissolution of free [6]LiI while preserving the encapsulated material, of the same nature, indicates that the ends/tips of the carbon nanomaterials became closed during the filling experiment. This hermetically sealed carbon system isolates the encapsulated compound from the external medium, which is an essential aspect for the development of CNC for biomedical purposes. It has been previously reported that the ends of MWCNTs can be closed by thermal annealing provided a high enough temperature is given to the system (Martincic M. et al. Carbon 2019, 141: 782-793). However, here also provide evidence that the tips of CNHs are also able to be regenerated by adequate thermal treatment.

Thermal regeneration of the ends of carbon nanostructures depends on their diameter. A higher temperature is for instance needed to seal multi-walled CNTs compared to their single-walled counterparts (Martincic M. et al. Carbon 2019, 141: 782-793). In the case of short-MWCNTs ,1100 °C were employed, whereas in the case of CNHs, 950 °C were established as the temperature to promote simultaneously the process of filling and end closing.

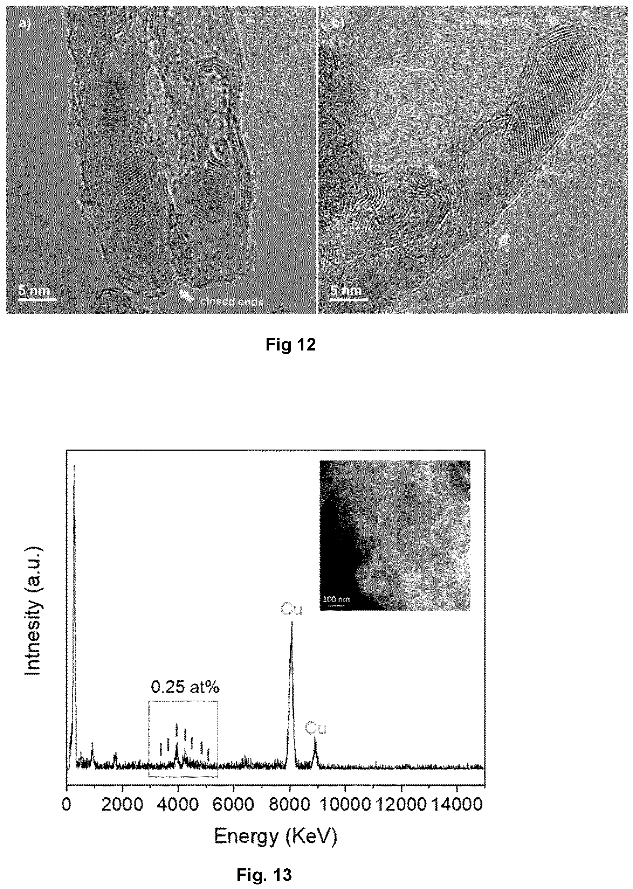

The successful formation of hermetically sealed nanostructures with [6]LiI nanometric crystals in the interior of MWCNTs and CNHs, was confirmed by transmission electron microscopy (Fig 2). Furthermore, it is also possible to confirm through visual inspection that the graphitic ends of the MWCNTs and CNHs (pointed with arrows) are reconstructed, forming in this way, the desired hermetically sealed CNCs (see Fig. 12 for additional images). The intensity profile acquired on the crystal observed in Fig 2c reveals an interplanar spacing of 3.47 Å. This is in good agreement with the (111) plane of LiI (Fm-3m (225), ICSD). It is interesting to notice that a tube-like contrast is observed in the inner cavities of some MWCNTs, which has been previously reported for other metal halides leading to the formation of tubular van der Waals heterostructures (Sandoval S. et al. ACS Nano 2018, 12(7): 6648-6656. Sandoval S. et al. Carbon 2017, 123: 129-134). Figure 2f shows a HRTEM image of a crystal confined inside a CNH. The intensity profile (inset) reveals an interplanar spacing of 3.01 Å, which is in agreement with the (200) plane of the LiI cubic structure.

Energy dispersive X-ray analysis (EDX) was next employed to confirm the presence of I and for the semi-quantitative determination of the atomic composition of the synthesized materials. An estimated concentration of 0.25 at. % I and 0.63 at. % I was determined for [6]LiI@MWCNTs and [6]LiI@CNHs, respectively (Fig. 13-14).

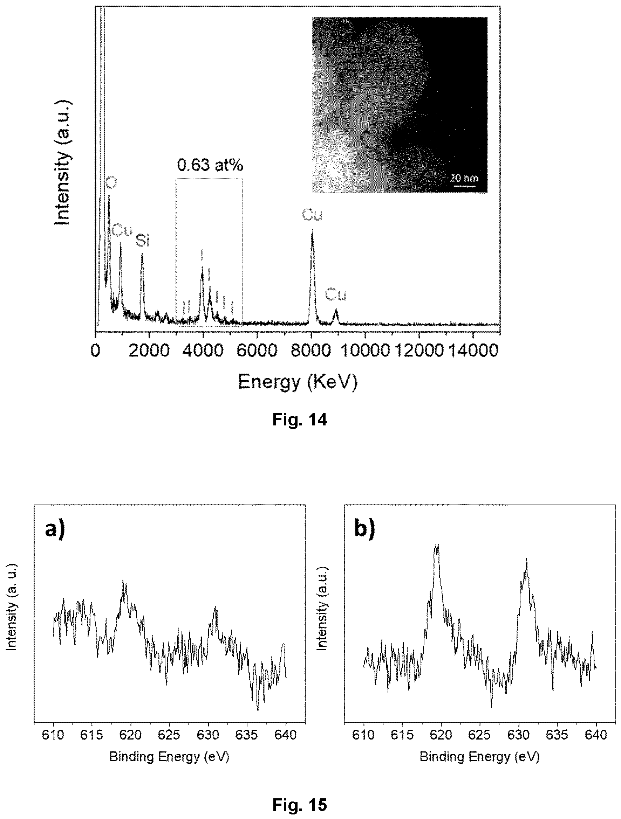

X-ray photoelectron spectroscopy (XPS) measurements are in agreement with the presence of I (from LiI) filled into both MWCNTs and CNHs cavities, with signals at ca. 620 eV corresponding to I3d (Fig. 15). Due to the very low atomic weight of lithium, its signal intensity was too low to be measurable by this technique. Therefore, the concentration of enriched lithium was determined based on the quantification of iodine in the $I3d_{5/2}$ high-resolution spectrum (Table 1). Considering that I and Li are equimolar (1:1 ratio), the estimated value for the atomic percentage of $^6Li$ in the sample is 1.4 at.% for $^6LiI@MWCNTs$ and 3.5 at. % for $^6LiI@CNHs$ (Table 1). Despite the $^6Li$ concentrations determined by EDX analyses (0.25 at. % and 0.63 at.% for $^6LiI@MWCNTs$ and $^6LiI@CNHs$, respectively; obtained also by indirect measurement through the iodine concentration) are much lower than those assessed by XPS, reveals a higher loading of Li in the cavities of CNHs than in MWCNTs.

**Table 1.** Sample composition, considering the atomic ratio obtained from the high resolution C1s, N1s and I3d XPS for the CNC before ($^6LiI@MWCNTs$ and $^6LiI@CNHs$) and after surface functionalization by cycloaddition reaction ($NH_2\_{}^6LiI@MWCNT$ and $NH_2\_{}^6LiI$ @CNH). H* and Li** content was determined based on the stoichiometry of both the aromatic moiety attached to the carbon surface ($C_6NH_6$) and the lithium iodide (1:1).

| Sample | Element (at. %) | | | | |
|---|---|---|---|---|---|
| | C | N | H* | I | Li** |
| $^6LiI@MWCNT$ | 97.2 | / | / | 1.4 | 1.4 |
| $^6LiI@CNH$ | 93 | / | / | 3.5 | 3.5 |
| $NH_2\_{}^6LiI@MWCNT$ | 85.1 | 1.9 | 11.6 | 0.7 | 0.7 |
| $NH_2\_{}^6LiI$ @CNH | 84.4 | 1.9 | 11.5 | 1.1 | 1.1 |

**Quantitative Neutron Autoradiography**

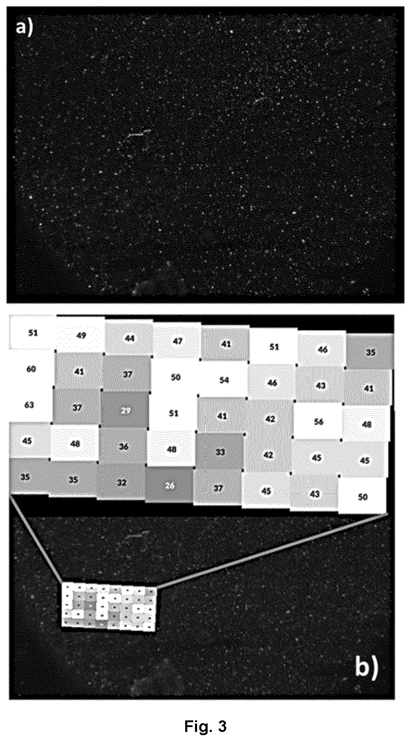

To test whether the developed $^6Li@CNC$ held potential for LiNCT an initial assessment was performed by neutron autoradiography using $^6LiI@MWCNTs$ dispersed in KBr pellets. $^6LiI@MWCNTs$ were chosen for these initial neutron irradiation trials because they have a lower content of $^6Li$ than $^6LiI@CNHs$. Therefore, if $^6Li$ nuclei reacting with neutrons are already detected the same would be expected for $^6LiI@CNHs$. Figure 3 shows the imaging of lithium distribution in KBr pellets. The grey-level maps show that alpha particles' tracks from neutron capture reaction on lithium are visible. It is important to notice that tracks are distributed over the entire pellet. Since MWCNTs tend to aggregate in bundles and the KBr pellet was prepared by simple grinding, the distribution of tracks is inhomogeneous. The inhomogeneities are shown by the white dots, which are track clusters given by lithium atoms aggregates. The quantitative neutron autoradiography analysis resulted in $44 \pm 8$ tracks mm$^{-2}$. This shows that the number of tracks in a portion of 12 mm$^2$ has a 20% variability due to the mild inhomogeneity. Remarkably, this analysis confirms the presence of active lithium species on the sample.

**External surface functionalization of CNC ($NH_2$-$^6Li@CNC$)**

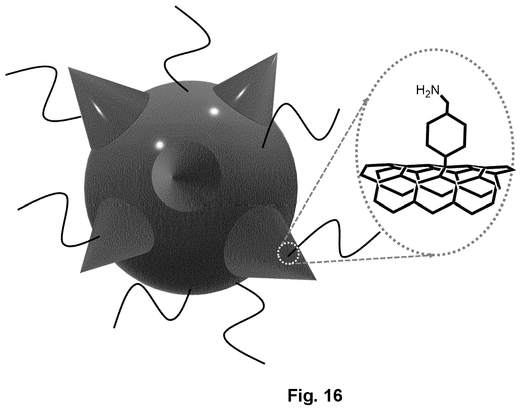

The external surface functionalization of the $CNC@^6LiI$ was performed using diazonium-based arylation reaction. Fig. 16 shows a schematic representation of the included functional groups. The presence of free amine groups on the external aromatic structure of the nanocarbon shelter, significantly improves its water dispersibility and it is expected to enhance its biocompatibility.

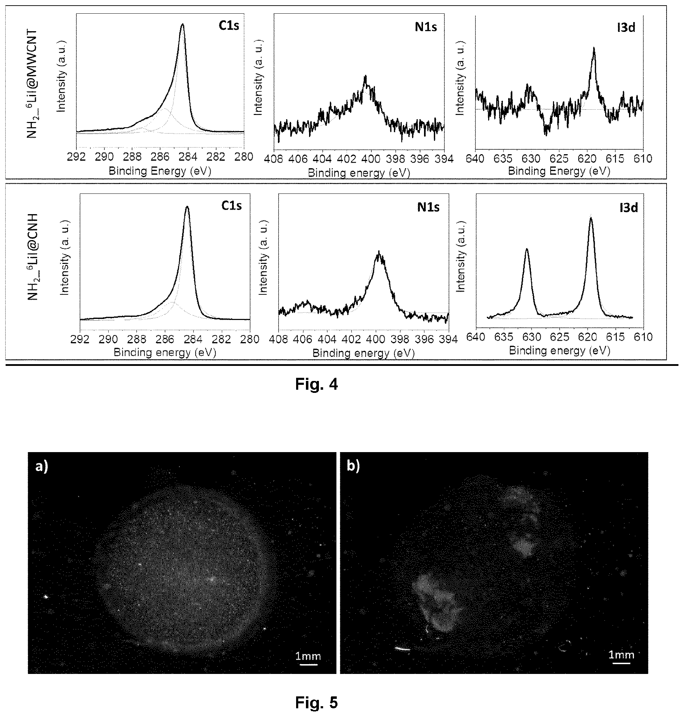

The composition of $^6LiI@CNC$ after chemical modification was determined by XPS analysis (Figure 4). High resolution XPS spectra of the C1s, N1s and I3d were employed to quantitatively assess the concentration of C, N and I in the amine functionalized samples. After considering the presence and stoichiometric ratio of both Li (from LiI) and H (present in the aminophenyl group), analyses reveal a similar relative atomic content of N1s for both $^6LiI@CNC$ (1.9 at. %). The amino loading was calculated taking into account the slight variations in C and I (the latter being higher in the case of $NH_2\_{}^6LiI@CNH$ (1.1 at. % of I) than $NH_2\_{}^6LiI@MWCNT$ (0.7 at. % of I)), the presence of H (6 H atoms per the amino group containing moiety) and the equimolar concentration of I and $^6Li$. This led to a surface chemical functionalization of 1.61 mmol and 1.67 mmol of -$NH_2$ per gram of CNHs and MWCNT, respectively. These results indicate that the yield of the cycloaddition reaction for the covalent surface modification of the employed carbon nanostructures is barely affected by their morphology.

**Qualitative Neutron Autoradiography**

Figures 6 show the qualitative neutron autoradiography of the $^6Li@CNC$ respectively produced by CNH and MWCNT. The image in Figure 5 a) shows that cells exposed to nanoparticles produced with CNH absorb lithium homogeneously, which is also confirmed from the quantitative analysis, which results in a mean track density of $23 \pm 5$ tracks mm$^{-2}$, where the uncertainty reflects the distribution in the sample. Low value of this uncertainty means that track density is very regular and does not fluctuate much between one sampled portion (0.3 mm$^2$ image) to another. On the other hand, lithium enriched nanoparticles produced by MWCNT are taken up in cells with poor uniformity (Figure 5 b)). This is also confirmed by the quantitative analysis which gives a mean track density of $20 \pm 20$ tracks mm$^{-2}$: 100 % uncertainty means that track density has high fluctuations from one sampled portion (0.3 mm$^2$ image) to another. This inhomogeneity can be due to the poor dispersibility of the compound in the cell culture medium and consequently a limited or no availability of lithium for the cells.

**Biocompatibility and neutron irradiation experiments**

For this test, only the promising $^6$Li@CNC compound produced with CNH was used for the neutron irradiation of cells to measure survival. Table 2 shows the obtained results evaluated with plating efficiency. When the cells are not irradiated but are enriched with lithium, the cell survival is 92 % resulting in a slight cytotoxicity, yet acceptable, effect of the nanocapsules. When cells are irradiated without lithium treatment, the survival drops at 4.2 % due to neutron interaction with hydrogen and nitrogen in cells (background dose). When cells are irradiated after treatment with lithiated compound, the survival is halved (1.8 %) due to effect of neutron interaction with lithium.

Table 2. Cell survival results obtained by plating efficiency

| $^6$Li enriched | Irradiated | Cell Survival (%) |
| --- | --- | --- |
| NO | NO | $100 \pm 10$ |
| Yes | NO | $92 \pm 12$ |
| NO | Yes | $4.2 \pm 0.3$ |
| Yes | Yes | $1.8 \pm 0.3$ |

Being an initial test, a high dose was employed to have a better chance to have a therapeutic effect. The use of such dose results in a high cell death in the absence of lithium, but remarkably, a 50 % decrease in cell survival is observed when the nanocapsules are present. With the present approach the encapsulated cargo remains biologically inactive until it is activated by an external neutron irradiation. This allows for the nanocapsules to be internalized by cells or even to be delivered to the diseased tissue in vivo with no biological effect. Only when the nanocapsules are located into the targeted site, become activated by the external neutron flux and turn lethal, thus behaving as carbon nuclear nanobombs (CNB). This contrasts to previous studies using radionuclides, where the nanocapsules delivering them are already administered in vivo in their radioactive form.

**Conclusions**

Nanocapsules for the containment of $^6$Li have been developed using both MWCNTs and CNHs. With neutron autoradiography, it has been shown that $^6$Li enters the UMR cells. The accumulation of $^6$Li in the cells is more homogeneous when using $^6$Li@CNC produced with CNH compared to that produced with MWCNT. In fact, in the first case the concentration of $^6$Li present in the cells produced an average spatial density of $23 \pm 5$ tracks mm$^{-2}$ in the CR39, while in the second case this was equal to $20 \pm 20$ tracks mm$^{-2}$. A first preliminary test of thermal neutron irradiation of UMR cells treated with $^6$Li@CNH showed that, with this concentration, the survival of the cells is reduced by 50 % compared to an irradiation without $^6$Li. In addition, the compound demonstrated low toxicity at the cellular level. These results give a clear indication of the possibility of using this compound for Lithium Neutron Capture Therapy (LiNCT) and encourage us to plan a systematic study that will allow us to estimate the dose in cells due to the reaction $^6_3Li(n, ^3_1H)^4_2He$ in order to produce a complete cell survival curve to evaluate the therapeutic potential of the developed nanocapsules. The present study opens the door to exploit the potential that LiNCT holds for the treatment of cancer.

**Claims**

1.    Carbon nanocapsule (CNC) encapsulating lithium isotope $^6$Li and wherein said nanocapsule is selected form a carbon nanohorns aggregate (CNHs) and closed-ended carbon nanotube (CNT).

2.    Carbon nanocapsule, according to claim 1, wherein said CNC is a closed-ended CNT selected from single-wall carbon nanotube (SWCNT) or multi-wall carbon nanotube (MWCNT).

3. Carbon nanocapsule, according to claim 2, wherein the length of the closed-ended CNT is between 80 and 300 nm, and the external diameter of said tube ranges from 5 to 30 nm in the case of MWCNT and from 1 to 2 nm in the case of SWCNT.

4. Carbon nanocapsule, according to claim 1, wherein said CNC is an aggregate of dahlia-like carbon nanohorns.

5. Carbon nanocapsule, according to claim 1 or 4, wherein the diameter of the CNHs ranges from 50 to 120 nm.

6. Carbon nanocapsule, according to any of previous claims, wherein the concentration of lithium isotope $^6$Li in the CNC ranges from 0.7 at. % to. 10 at. %.

7. Carbon nanocapsule, according to any of previous claims, wherein CNC comprises no other neutron capture element than $^6$Li.

8. Carbon nanocapsule, according to any of previous claims, wherein, lithium isotope $^6$Li is in the form of a lithium salt selected from: $^6$LiCl, $^6$LiF, $^6$LiI, $^6$LiNO$_3$, $^6$Li$_2$SO$_4$ and $^6$Li$_2$CO$_3$.

9. Carbon nanocapsule, according to any of previous claims, wherein the external surface of the CNC is functionalized with a group selected from: amine, carboxylic acid, hydroxyl, dextran, pluronic acid, polyethylene glycol (PEG) and albumin.

10. Carbon nanocapsule, according to claim 9, wherein the external surface of the CNC is functionalized with free amine groups -NH$_2$.

11. Carbon nanocapsule, according to claim 9 or 10, wherein the groups that functionalize the surface ranges from 1 to 8 mmol·g$^{-1}$.

12. A process for preparing CNCs described in any of the claims 1 to 11 that comprises, from CNCs having open ends/tips, the encapsulation of $^6$Li and closing their opened ends/tips by annealing a mixture of CNCs with a $^6$Li salt in a weight ratio CNCs: $^6$Li salt from 1:5 to 1:20 at a temperature between 700 and 1300 °C for 4-12h.

13. The process, according to claim 12, wherein the annealing in step is carried out in a weight ratio CNCs: $^6$Li salt of 1:10.

14. The process, according to claim 12 or 13, wherein the CNCs are CNTs, and the process comprises, before the encapsulation and closing step, the steps of:

- oxidation of closed-ended CNTs to open their ends by treating them with a strong oxidizing medium: nitric acid ora mixture of nitric:sulphuric acids for 6-15 h.
- thermal reduction of the CNTs, obtained in the previous step, at 500-800 °C for 1-2 h under vacuum.

15. The process, according to claim 12 or 13, wherein the CNCs are CNHs, and the process comprises, before the encapsulation and closing step, the step of:
oxidation of the CNHs to open their tips by annealing them up to 400-550 °C under air.

16. The process, according to any of claims 12 to 15, that comprises, after encapsulation and closing step, a further step of external surface functionalization of the CNC with free amine groups -NH$_2$ by diazonium-based arylation reaction.

17. Carbon nanocapsule, according to any of claims 1 to 11, for use as a medicament.

18. Carbon nanocapsule, according to any of claims 1 to 11, for use in the diagnosis or treatment of cancer.

19. Carbon nanocapsule, according to any of claims 1 to 11, for use for use in neutron capture therapy (NCT).

20. Carbon nanocapsule, according to any of claims 1 to 11, for use in neutron capture enhanced particle therapy (NCEPT).

Fig. 1

FIG. 2

Fig. 3

**Fig. 4**

**Fig. 5**

**Fig. 6**

**Fig. 7**

Fig. 8

Fig. 9

**Fig. 10**

**Fig. 11**

**Fig 12**

**Fig. 13**

**Fig. 14**

**Fig. 15**

Fig. 16

## INTERNATIONAL SEARCH REPORT

| | International application No |
|---|---|
| | PCT/ES2023/070187 |

**A. CLASSIFICATION OF SUBJECT MATTER**
INV.   C01B32/178     C01B32/18      A61K51/02      A61P35/00
ADD.

According to International Patent Classification (IPC) or to both national classification and IPC

**B. FIELDS SEARCHED**

Minimum documentation searched  (classification system followed by classification symbols)
C01B   A61K   A61P

Documentation searched other than minimum documentation to the extent that such documents are included  in the fields searched

Electronic data base consulted during the  international search (name of data base and,  where practicable, search terms used)

EPO-Internal, WPI Data

**C. DOCUMENTS CONSIDERED TO BE RELEVANT**

| Category* | Citation of document, with indication,  where appropriate, of the relevant passages | Relevant to claim No. |
|---|---|---|
| X | BROWN G. ET AL: "High yield incorporation and washing properties of halides incorporated into single walled carbon nanotubes", APPLIED PHYSICS A, vol. 76, no. 4, 1 March 2003 (2003-03-01), pages 457-462, XP093057494, Berlin/Heidelberg ISSN: 0947-8396, DOI: 10.1007/s00339-002-2040-1 Retrieved from the Internet: URL:http://link.springer.com/article/10.10 07/s00339-002-2040-1/fulltext.html> table 1 ----- | 1-20 |
| A | GB 2 383 534 A (PSIMEI PHARMACEUTICALS PLC [GB]) 2 July 2003 (2003-07-02) claims 1, 8 ----- | 1-20 |

-/--

| ☒ Further documents are listed in the  continuation of Box C. | ☒ See patent family annex. |
|---|---|

* Special categories of cited documents :

"A" document defining the general state of the art which is not considered to be of particular relevance

"E" earlier application or patent but published on or after the international filing date

"L" document which may throw doubts on priority  claim(s) or which is cited to establish the publication date of another  citation or other special reason (as specified)

"O" document referring to an oral disclosure, use,  exhibition or other means

"P" document published prior to the international filing date but later than the priority date claimed

"T" later document published after the international filing date or priority date and not in conflict with the application but cited to understand the principle or theory underlying the invention

"X" document of particular relevance;; the claimed invention cannot be considered novel or cannot be considered to involve an inventive step when the document is taken alone

"Y" document of particular relevance;; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 27 June 2023 | 05/07/2023 |

| Name and mailing address of the ISA/ European Patent Office, P.B. 5818 Patentlaan 2 NL - 2280 HV Rijswijk Tel. (+31-70) 340-2040, Fax: (+31-70) 340-3016 | Authorized officer Mattheis, Chris |
|---|---|

Form PCT/ISA/210 (second sheet) (April 2005)

## INTERNATIONAL SEARCH REPORT

International application No

**PCT/ES2023/070187**

**C(Continuation). DOCUMENTS CONSIDERED TO BE RELEVANT**

| Category* | Citation of document, with indication, where appropriate, of the relevant passages | Relevant to claim No. |
|---|---|---|
| A | MARTINCIC MARKUS ET AL: "Non-cytotoxic carbon nanocapsules synthesized via one-pot filling and end-closing of multi-walled carbon nanotubes", CARBON, ELSEVIER OXFORD, GB, vol. 141, 5 October 2018 (2018-10-05), pages 782-793, XP085537118, ISSN: 0008-6223, DOI: 10.1016/J.CARBON.2018.10.006 Experimental ----- | 1-20 |

Form PCT/ISA/210 (continuation of second sheet) (April 2005)

page 2 of 2

**INTERNATIONAL SEARCH REPORT**

Information on patent family members

| | International application No |
|---|---|
| | PCT/ES2023/070187 |

| Patent document cited in search report | | Publication date | Patent family member(s) | | Publication date |
|---|---|---|---|---|---|
| GB 2383534 | A | 02-07-2003 | AT | 477818 T | 15-09-2010 |
| | | | AU | 2002356328 A1 | 15-07-2003 |
| | | | EP | 1471939 A1 | 03-11-2004 |
| | | | ES | 2350975 T3 | 28-01-2011 |
| | | | GB | 2383534 A | 02-07-2003 |
| | | | US | 2005180917 A1 | 18-08-2005 |
| | | | WO | 03055520 A1 | 10-07-2003 |

Form PCT/ISA/210 (patent family annex) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MALOUFF T. et al.** *Frontiers in Oncology*, 2021, vol. 11, 601820
- **HIRATSUKA J. et al.** *Cancer Communications*, 2018, vol. 38 (1), 38
- **DYMOVA MA et al.** *Cancer Communications*, 2020, vol. 40, 406
- **HE H et al.** *Radiation Oncology*, 2021, vol. 16, 216
- **BARTH RF. et al.** *Cancer Communications*, 2018, vol. 38 (1), 35
- **NAKAMURA H. et al.** *Bioorganic and Medicinal Chemistry Letters*, 2015, vol. 25 (2), 172-174
- **SUMITANI S ; NAGASAKI Y.** *Polymer Journal*, 2012, vol. 44 (6), 522-530
- **KOGANEI H. et al.** *Bioconjugate Chemistry*, 2013, vol. 24 (1), 124-132
- **ALBERTI D. et al.** *Organic and Biomolecular Chemistry*, 2014, vol. 12 (15), 2457-2467
- **ENGER SA et al.** *Radiation Measurements*, 2013, vol. 59, 233-240
- **MARTIN RF et al.** *Pigment Cell Research*, 1989, vol. 2 (4), 330-332
- **DE STASIO G. et al.** *Neurological Research*, 2005, vol. 27 (4), 387-398
- **HO SL et al.** *RSC Advances*, 2018, vol. 8 (23), 12653-12665
- **SINNOTT S. et al.** *Critical Reviews in Solid State and Materials Sciences*, 2001, vol. 26, 145
- **LIU X. et al.** *Biosensors and Bioelectronics*, 2020, vol. 167, 112495

- **GEORGAKILAS V. et al.** *Journal of the American Chemical Society*, 2002, vol. 124 (5), 760-761
- **KIERKOWICZ M. et al.** *Carbon*, 2018, vol. 139, 922-932
- **UTSUMI S et al.** *The Journal of Physical Chemistry B*, 2005, vol. 109 (30), 14319-14324
- **BROWN G. et al.** *Applied Physics A*, 2003, vol. 76 (4), 457-462
- **MARTINCIC M. et al.** *Carbon*, 2019, vol. 141, 782-793
- **BORTOLUSSI S. et al.** *Radiation Oncology*, 2017, vol. 12 (1), 130
- **POSTUMA I. et al.** *Reports of Practical Oncology & Radiotherapy*, 2016, vol. 21 (2), 123-128
- **ALTIERI S. et al.** *Applied Radiation and Isotopes*, 2008, vol. 66 (12), 1850-1855
- **SERPELL CJ. et al.** *Nature Communications*, 2016, vol. 7, 13118
- *J. Phys. Chem. B*, 2006, vol. 110 (4), 1587-1591
- **WANG JT-W. et al.** *Carbon*, 2020, vol. 162, 410-422
- **MARTINCIC M. ; TOBIAS G.** *Expert Opinion on Drug Delivery*, 2015, vol. 12 (4), 563-581
- **KIERKOWICZ M. et al.** *ACS Sustainable Chemistry & Engineering*, 2017, vol. 5 (3), 2501-2508
- **SANDOVAL S. et al.** *ACS Nano*, 2018, vol. 12 (7), 6648-6656
- **SANDOVAL S. et al.** *Carbon*, 2017, vol. 123, 129-134